(19) Europäisches Patentamt
European Patent Office
Office européen des brevets

(11) Veröffentlichungsnummer : **0 053 331**
**B1**

# (12) EUROPÄISCHE PATENTSCHRIFT

(45) Veröffentlichungstag der Patentschrift : **13.03.85**

(51) Int. Cl.⁴ : **C 07 C143/828**

(21) Anmeldenummer : **81109792.2**

(22) Anmeldetag : **20.11.81**

## (54) Kontinuierliches Verfahren zur Herstellung von Chloralkylsulfonylisocyanaten.

(30) Priorität : **28.11.80 DE 3044980**

(43) Veröffentlichungstag der Anmeldung : **09.06.82 Patentblatt 82/23**

(45) Bekanntmachung des Hinweises auf die Patenterteilung : **13.03.85 Patentblatt 85/11**

(84) Benannte Vertragsstaaten : **AT BE CH DE FR GB LI NL**

(56) Entgegenhaltungen :
**DE-B- 1 226 565**
**DE-B- 1 230 016**

(73) Patentinhaber : **HOECHST AKTIENGESELLSCHAFT**
**Postfach 80 03 20**
**D-6230 Frankfurt am Main 80 (DE)**

(72) Erfinder : **Wegener, Peter, Dr.**
**Am Eichkopf 4**
**D-6240 Königstein/Taunus (DE)**
Erfinder : **Riemenschneider, Wilhelm, Dr.**
**Loreleistrasse 45**
**D-6230 Frankfurt am Main 80 (DE)**
Erfinder : **Ulmschneider, Dieter, Dr.**
**Im Flemetz 6**
**D-6240 Königstein/Taunus (DE)**

EP 0 053 331 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Ein Verfahren zur Herstellung von Chloralkylsulfonylisocyanaten durch katalytisch induzierte Reaktion zwischen Olefinen und Chlorsulfonylisocyanat ist bereits aus der DE-PS 1 211 165 bekannt.

Die Reaktion verläuft drucklos und ist in ihrem Raum-Zeit-Umsatz durch die Abführung der Reaktionswärme begrenzt. (D. Günther, F. Soldan, Chem. Ber. *103,* 663 (1970) und Angew. Chem. 80, S. 187 (1968)). Führt man die Reaktion in größeren Ansätzen aus, so treten erhebliche Schwankungen in Ausbeute und Umsatz auf, die auch durch die üblichen Maßnahmen wie verstärktes Rühren, verbesserte Wärmeabführung und Katalysator-Dosierung nicht behoben werden können.

Es stellte sich daher die Aufgabe, diesen unsicheren Reaktionsablauf zu überwinden und ein Verfahren zu entwickeln, das die Herstellung der gewünschten Substanzen auch in größerem, technischen Maßstab mit gleichbleibend guter Ausbeute und Umsatz gestattet.

Zunächst wurden Rieselsäulen mit Füllkörpern und eine Blasensäule als Reaktoren untersucht, die jedoch in Umsatz und Ausbeute weit hinter dem absatzweisen Verfahren zurückblieben. Insbesondere stieg hierbei der Gehalt des als Nebenprodukt unerwünschten 1 : 2-Adduktes nämlich 3-Oxo-2-[2-chlorethyl]-isothiazolidin-1,1-dioxid, relativ zum gewünschten 1 : 1-Addukt bis über 20 % an, bei der Blasensäule erfolgte auch eine Rück- und Quermischung des Reaktionsstromes, die eine gleichmäßige und kontrollierte örtliche und zeitliche Katalysatorkonzentration unmöglich machte.

Überraschenderweise gelang es nun, dieses Nebenprodukt stark zu verringern, die Reaktion exakt zu kontrollieren und mit hoher Ausbeute durchzuführen, indem man die Reaktion kontinuierlich betreibt und als Reaktionsgefäß ein langes Rohr verwendet, in dem Chlorsulfonylisocyanat, das Olefin und der Katalysator eingeführt und zur Reaktion gebracht werden.

Gegenstand der Erfindung ist somit ein Verfahren zur Herstellung von Chloralkylsulfonylisocyanaten der Formel

$$\begin{array}{l} Cl - CH - CH_2 - SO_2NCO \\ \phantom{Cl - }\,| \\ \phantom{Cl - }R \end{array}$$

wobei R Wasserstoff, Methyl oder Ethyl bedeutet, durch katalytisch induzierte Reaktion von Chlorsulfonylisocyanat mit einem oder mehreren $C_2$-$C_4$-Olefinen, dadurch gekennzeichnet, daß man die Reaktion kontinuierlich in einem Röhrenreaktor durchführt.

Als Katalysatoren kommen alle Verbindungen in Frage, die in der Lage sind, unter den Reaktionsbedingungen Radikale zu erzeugen, wie beispielsweise Peroxide oder Azoverbindungen. Im einzelnen seien genannt Azo- bis-isobutyronitril, Dialkyl-peroxydicarbonate, oder Benzoylperoxid. Der Katalysator wird im allgemeinen in Mengen von 0,01 bis 20 Gew.-% bezogen auf Chlorsulfonylisocyanat, eingesetzt. Die gesamte Menge an Katalysator kan am Anfang des Reaktors auf einmal zugegeben werden, man kann aber auch die gesamte Menge des Katalysators aufteilen und diese Teilmengen, die verschieden groß sein können, an unterschiedlichen Stellen in den Röhrenreaktor eingeben. Olefin und Chlorsulfonylisocyanat können im Gleichstrom oder im Gegenstrom durch den Reaktor geführt werden ; die Arbeitsweise im Gleichstrom ist bevorzugt.

Diese Art der Zugabe des Katalysators bedingt, im Gegensatz zu der vorbekanten Arbeitsweise, wo während der gesamten Reaktion durch gleichmäßige Zugabe eine konstante Konzentration an Katalysator aufrechterhalten wird, daß hier die Konzentration an Katalysator im Verlauf der Reaktion stark schwankt. Es war deshalb überraschend zu finden, daß man bei der kontinuierlichen Arbeitsweise trotzdem gleichgute oder sogar höhere Ausbeuten erhält im Vergleich zur diskontinuierlichen Arbeitsweise.

Die Reaktion wird bei erhöhter Temperatur ausgeführt, wobei sich ein Bereich zwischen 60 und 80° als vorteilhaft herausgestellt hat. Die für den Einzelfall optimale Temperatur richtet sich in erster Linie nach dem verwendeten Katalysator bzw. dessen Zerfallstemperatur.

Bei dem zur Durchführung der Reaktion benutzten Röhrenreaktor soll das Verhältnis von Durchmesser zur Länge zwischen 1 : 100 und 1 : 10 000 betragen.

Bevorzugt ist ein Durchmesser- zur Längenverhältnis von 1 : 500 bis 1 : 4 000. Die Verweilzeit im Rohr beträgt optimal 1-3 Stunden, jedoch sind auch kürzere Zeiten bis zu 1/2 Stunden und längere Zeiten bis zu 8 Stunden möglich. Der Druck in dem Röhrenreaktor beträgt zwischen 1 und 5 bar. Die Verwendung eines Lösungs- oder Verdünnungsmittels ist bei diesem Verfahren nicht erforderlich.

Es ist ohne weiteres möglich, nicht nur ein, sondern auch zwei oder drei verschiedene Olefine mit dem Chlorsulfonylisocyanat zur Reaktion zu bringen. Das kann entweder durch gleichzeitiges oder nacheinanderfolgendes Einleiten der Olefine erfolgen. Auf jeden Fall erhält man dabei eine Mischung der entsprechenden Chloralkylsulfonylisocyanate.

Vorteilhaft bei der Durchführung der Reaktion ist ein gewisser Überschuß von ca. 10 % des eingesetzten Olefins über die stöchiometrisch benötigte Menge hinaus. Unter diesen Bedingungen wird mit Ethylen eine Selektivität von ca. 85 % bei 50 % Umsatz erhalten, mit Propylen bei gleicher Selektivität über 90 % Umsatz. Der Anteil an 2 : 1-Addukt liegt dabei unter 5 %.

Die erfindungsgemäß hergestellten Chlorsulfonylisocyanate sind wertvolle Zwischenprodukte. So kann man daraus beispielsweise durch Umsetzung mit Stearylamin ein Lederhilfsmittel herstellen.

Der Vorteil des erfindungsgemäßen Verfahrens besteht darin, daß die Reaktion in einer völlig geschlossenen Apparatur mit kleinem Reaktorvolumen durchgeführt wird. Dadurch kann der Umgang mit den sehr feuchtigkeitsempfindlichen Isocyanaten sicherer gestaltet und eine Belästigung des Bedienungspersonals stark gesenkt werden. Der Wegfall des Verdünnungsmittels und die Reduzierung des Druckes bedeuten außerdem weitere technische Vorteile gegenüber dem bisher angewandten Verfahren.

Beispiel 1-7

(siehe Tabelle 1)

Ein Schlauch aus Polytetrafluorethylen (PTFE) oder ein Edelstahl-(V4A)-Rohr mit 5 bzw. 10 mm Innendurchmesser und 4, 10 bzw. 20 m Länge wird, zur Rolle aufgewicklet, in einem Heizbad erhitzt. Der Katalysator wird im Chlorsulfonylisocyanat gelöst und zusammen mit diesem eingepumpt. Ethylen wird am Rohreingang eingepreßt, der Druck über ein Reduzierventil eingestellt. Entleert wird das Produkt über ein Drosselventil am Schlauchende. Die Analysenwerte sind gaschromatografisch ermittelte Flächen-Prozente und entsprechen etwa den Gewichtsprozenten.

(Siehe Tabelle 1 Seite 4 f.)

Tabelle 1

| Beispiel | Rohrabmessungen | | | | | CSI | Katalysator-Konzentration | Verw. Zt. | Temp. | Druck | Flächen-% nach GC-Analyse | |
|---|---|---|---|---|---|---|---|---|---|---|---|---|
| | Ø mm | Länge mm | Material | Füllung | $\frac{Ø}{Länge}$ | $\frac{ml}{h}$ | Mol % | h | °C | bar | Clesi % | 2:1 Add. % |
| 1 | 5 | 10000 | PTFE | 50 | $\frac{1}{2000}$ | 25 | IPPC 0,35 | 2 | 75 | 2 | 39 | 8 |
| 2 | 5 | 10000 | V4A | 50 | " | 50 | " | 1 | 75 | 2 | 31 | 3 |
| 3 | 5 | 10000 | PTFE | 50 | " | 50 | EHPC 0,35 | 1 | 75 | 2 | 56 | 10 |
| 4 | 5 | 20000 | " | 100 | $\frac{1}{4000}$ | 50 | NBPC 0,35 | 2 | 75 | 2 | 57 | 7 |
| 5 | 5 | 10000 | V4A | 50 | $\frac{1}{2000}$ | 50 | IPPC 0,35 | 1 | 75 | 16 | 25 | 13 |
| 6 | 10 | 4000 | " | 100 | $\frac{1}{400}$ | 100 | " | 1 | 75 | 2 | 10 | 1,7 |
| 7 | 10 | 4000 | " | 100 | " | 50 | " | 2 | 65 | 8 | 16 | 10 |

CSI = Chlorsulfonylisocyanat
IPPC = Isopropyl-Peroxidicarbonat
EHPC = Ethylhexyl-Peroxidicarbonat
NBPC = n-Butyl-Peroxidicarbonat
Clesi = Chlorethylsulfonylisocyanat
PTFE = Polytetrafluorethylen

## Beispiel 8-13

(siehe Tabelle 2)

Eine Apparatur bestehend aus einer Glasschlange von ca. 1 cm lichter Weite und etwa 9 m Länge mit einem Volumen von ca. 7 l wird mit umlaufendem Öl auf 70 °C geheizt. Chlorsulfonylisocyanat und der Katalysator wurden getrennt eingepumpt, Ethylen oder Propylen im Gleichstrom über ein Druckhalteventil zugeführt. Die Katalysatorzugabe erfolgte in zwei Teilmengen am Eingang des Reaktors unten und in der Mitte. Das Reaktionsprodukt wurde am oberen Reaktorausgang entspannt und in einem Abscheider von überschüssigen Olefin getrennt. Das Chloralkylsulfonylisocyanat fließt unten aus dem Abscheider ab und kann je nach Verwendungszweck in der angefallenen Form weiter umgesetzt oder durch Destillation gereinigt werden. Die Analysen wurden in der Mitte und am Reaktorausgang oben genommen. Die Analysenwerte stellen gaschromatographisch ermittelte Flächenprozente dar und entsprechen etwa den Gewichtsprozenten.

Beispiel 10 zeigt, daß bei Gegenstrom von Gas und Flüssigkeit keine besseren Ergebnisse als bei Gleichstrom erhalten werden.

## Beispiel 14 und 15

In dem in den Beispielen 8-13 beschriebenen Röhrenreaktor wurde an der unteren Eingabestelle wie im Beispiel 9 pro Stunde 1,0 kg Chlorsulfonylisocyanat, 10 g n-Butyl-Peroxidicarbonat als Katalysator und Ethylen unter einem Druck von 2 bar eingespeist, die Temperatur betrug 70 °C. In der Mitte des Reaktors wurde Propylen unter 2 bar Druck und nochmals 10 g n-Butyl-Peroxidicarbonat-Katalysator eingegeben. Die Zusammensetzung des Reaktionsgemisches am Ausgang wurde wie folgt analysiert :

7,2 % Chlorsulfonylisocyanat, 41,3 % Chlorethylsulfonylisocyanat.
44,1 % Chlorpropylsulfonylisocyanat und 3,9 % 2 : 1 Addukt.

Bei gleichzeitiger Zugabe von Ethylen und Propylen unter 2 bar am Eingang des Reaktors erhält man folgende Zusammensetzung des Gemisches am Ausgang :

5,3 % Chlorsulfonylisocyanat ; 19,3 % Chlorethylsulfonylisocyanat ; 67 % Chlorpropylsulfonylisocyanat und 3,7 % 2 : 1 Addukt.

## Beispiel 16

In dem in den vorhergehenden Beispielen beschriebenen Röhrenreaktor wurde analog Buten-1 unter 1 bar mit 2 kg/h Chlorsulfonylisocyanat und 2 × 10 g/h n-Butyl-Peroxidicarbonat als Katalysator umgesetzt, Temperatur 70 °C. Der Austrag hatte folgende Zusammensetzung :

43,6 % Chlorsulfonylisocyanat ; 37,3 % 2-Chlorbutansulfonylisocyanat und 1,9 % 2 : 1-Addukt.

(Siehe Tabelle 2 Seite 6 f.)

Tabelle 2

Ethylen + CSI

| Beispiel | Strömungs-richtung | CSI kg/h | Katalys. NBPC g/h | Temp. °C | Druck bar | Vers.Dauer h | Gesamt-Einsatz CSI kg | Probe-nahme | Flächen-% nach GC-Analyse CSI % | Clesi % | 2:1-Addukt % |
|---|---|---|---|---|---|---|---|---|---|---|---|
| 8/1 | gleich | 3,0 | 2 x 20 | 70 | 2 | 5 | 16,0 | Mitte | 52,4 | 36,5 | 2,2 |
| /2 | | | | | | | | oben | 45,4 | 44,1 | 2,3 |
| /3 | " | 3,0 | 2 x 20 | 70 | 2 | 6 | 19,0 | oben | 51,2 | 39,7 | 2,2 |
| 9/1 | " | 1,8-2,0 | 2 x 10 | 70 | 2 | 7 | 13,0 | Mitte | 52,6 | 37,9 | 4,9 |
| /2 | | | | | | | | oben | 45,6 | 45,1 | 4,5 |
| 10/1 | gegen | 1,8-2,0 | 2 x 10 | 70 | 2 | 6 | 13,0 | Mitte | 74,7 | 19,0 | 1,7 |
| /2 | | | | | | | | unten | 57,3 | 33,0 | 4,3 |
| Propylen + CSI | | | | | | | | | | Me-Clesi | |
| 11/1 | gleich | 3,0 | 40 | 70 | 2 | 5,5 | | Mitte | 19,1 | 72,8 | 1,5 |
| /2 | | | | | | | | oben | 7,8 | 83,0 | 1,3 |
| 12/1 | " | 2,0 | 40 | 70 | 2 | 6 | | Mitte | 4,0 | 83,4 | 2,6 |
| /2 | | | | | | | | oben | 0,9 | 87,2 | 3,1 |
| 13/1 | " | 2,0 | 40 | 70 | 2 | 46,5 | 95,5 | Mitte | 15,8 | 75,5 | 1,7 |
| /2 | | | | | | | | oben | 6,3 | 81,8 | 3,6 |
| /3 | " | | " | " | " | " | " | Mitte | 19,3 | 72,5 | 1,4 |
| /4 | | | | | | | | oben | 0,4 | 81,3 | 2,8 |
| /5 | " | " | " | " | " | " | " | Mitte | 11,7 | 75,8 | 2,4 |
| /6 | | | | | | | | oben | 7,0 | 83,9 | 2,7 |

Me-Clesi = β-Chlorpropylsulfonylisocyanat

**Ansprüche**

1. Verfahren zur Herstellung von Chloralkylsulfonylisocyanaten der Formel

$$Cl - \underset{\displaystyle R}{\underset{|}{CH}} - CH_2 - SO_2NCO$$

wobei R Wasserstoff, Methyl oder Ethyl bedeutet, durch katalytisch induzierte Reaktion von Chlorsulfonylisocyanat mit einem oder mehreren $C_2$-$C_4$-Olefinen, dadurch gekennzeichnet, daß man die Reaktion bei erhöhter Temperatur und einem Druck von 1 bis 5 bar kontinuierlich in einem Röhrenreaktor durchführt, bei dem das Verhältnis von Durchmesser zur Länge der Röhren 1 : 100 bis 1 : 10 000 beträgt.

2. Verfahren nach Anspruch 1, dadurch gekennzeichnet, daß das Verhältnis von Durchmesser zur Länge der Röhren 1 : 500 bis 1 : 4 000 beträgt.

3. Verfahren nach Anspruch 1, dadurch gekennzeichnet, daß die Katalysatoreingabe an mehreren Punkten des Röhrenreaktors gegebenenfalls in verschiedenen Mengen erfolgt.

4. Verfahren nach Anspruch 1, dadurch gekennzeichnet, daß bei Temperaturen zwischen 60 und 80° gearbeitet wird.

5. Verfahren nach Anspruch 1-4, dadurch gekennzeichnet, daß Olefine und Chlorsulfonylisocyanat im Gleichstrom durch den Reaktor geleitet werden.

6. Verfahren nach Anspruch 1, dadurch gekennzeichnet, daß Ethylen und Propylen entweder gleichzeitig oder nacheinander in den Reaktor eindosiert werden.

**Claims**

1. A process for the production of chloroalkylsulfonyl isocyanates of the formula

$$Cl - \underset{\displaystyle R}{\underset{|}{CH}} - CH_2 - SO_2NCO$$

in which R denotes hydrogen, methyl or ethyl, by a catalytically induced reaction of chlorosulfonyl isocyanate with one or more $C_2$-$C_4$-olefins, which comprises carrying out the reaction at an elevated temperature and at a pressure of 1 to 5 bar in a continuous manner in a tubular reactor for which the ratio of diameter to length of the tubes is 1 : 100 to 1 : 10,000.

2. A process as claimed in claim 1, wherein the reaction is carried out in a tubular reactor for which the ratio of diameter to length of the tubes is 1 : 500 to 1 : 4,000.

3. A process as claimed in claim 1, wherein the catalyst is introduced into the tubular reactor at several points, as appropriate in differing amounts.

4. A process as claimed in claim 1, wherein the reaction is carried out at temperatures between 60 and 80 °C.

5. A process as claimed in claim 1-4, wherein olefins and chlorosulfonyl isocyanate are passed through the reactor co-currently.

6. A process as claimed in claim 1, wherein ethylene and propylene are metered into the reactor either simultaneously or successively.

**Revendications**

1. Procédé de préparation de chloralkylsulfonylisocyanates répondant à la formule

$$Cl - \underset{\displaystyle R}{\underset{|}{CH}} - CH_2 - SO_2NCO$$

dans laquelle R désigne un atome d'hydrogène ou un groupe méthyle ou éthyle, par une réaction amorcée catalytiquement du chlorosulfonylisocyanate avec une ou plusieurs oléfines en $C_2$-$C_4$, caractérisé en ce qu'on effectue la réaction à chaud et sous une pression de 1 à $5.10^5$ Pa en continu dans un réacteur tubulaire, dans lequel le rapport du diamètre à la longueur des tubes est de 1 : 100 à 1 : 10 000.

2. Procédé suivant la revendication 1, caractérisé en ce que le rapport du diamètre à la longueur des tubes est de 1 : 500 à 1 : 4 000.

3. Procédé suivant la revendication 1, caractérisé en ce que l'introduction du catalyseur s'effectue en plusieurs points du réacteur tubulaire, le cas échéant en quantités différentes.

4. Procédé suivant la revendication 1, caractérisé en ce qu'on opère à des températures entre 60 et 80 °C.

5. Procécé suivant les revendications 1-4, caractérisé en ce que les oléfines et le chlorosulfonylisocyanate sont envoyés à travers le réacteur en écoulements cocourants.

6. Procédé suivant les revendications 1-4, caractérisé en ce que l'éthylène et le propylène sont introduits en quantités dosées soit simultanément soit successivement dans le réacteur.